# EUROPEAN PATENT SPECIFICATION

(11) **EP 0 347 000 B1**
(45) Date of publication and mention of the grant of the patent: **26.10.1994**
(21) Application number: 89201543.9
(22) Date of filing: 13.06.1989
(51) Int. Cl.: A61K 31/165, A61K 31/00

(54) **The use of vanilloids for the manufacture of a medicament for treating herpes simplex infections**
Anwendung von Vanillin-Derivaten zur Herstellung eines Arzneimittels zur Behandlung von Herpes simplex-Infektion
Utilisation de dérivés de la vanilline pour la fabrication d'un médicament utile dans le traitement de l'infection de l'herpes simplex

(30) Priority: 17.06.1988 US 208321; 02.06.1989 US 358751
(43) Date of publication of application: 20.12.1989
(73) Proprietor: THE PROCTER & GAMBLE COMPANY, Cincinnati, Ohio 45202 (US)
(72) Inventor: O'Neill, Timothy Peter, Wyoming Ohio 45215 (US); Kasting, Gerald Bruce, Wyoming Ohio 45215 (US); Cupps, Thomas Lee, Oxford Ohio 45056 (US)
(74) Representative: Engisch, Gautier

(56) References cited:
- EP-A- 0 149 545
- EP-A- 0 282 127
- US-A- 4 313 958
- US-A- 4 536 404
- THE JOURNAL OF INFECTIOUS DISEASES, vol. 162, no. 1, July 1990, pages 29-34,The University of Chicago Press; L. R. STANBERRY: "Capsaicin interferes withthe centrifugal spread of virus in primary and recurrent genital herpes simplexvirus infections"
- JOURNAL OF THE NEUROLOGICAL SCIENCES; vol. 72, 1986, pages 223-230, ElsevierSicence Publishers B.V. (Biomedical Division); A. LJUNGDAHL et al.: "Herpessimplex virus infection in capsaicin-treated mice"
- JOURNAL OF THE AMERICAN ACADEMY OF DERMATOLOGY, vol. 17, no. 1, 1987, pages 93-96, American Academy of Dermatology, Inc.; J. E. BERNSTEIN et al.: "Treatmentof chronic postherpetic neuralgia with topical capsaicin"
- R. HULL et al.: "VIROLOGY", 'Directory & Dictionary of Animal,Bacterial and Plant Viruses', 1989, Macmillan Reference books,London, GB;

## Description

This application relates to the use of vanilloid compounds for the treatment or prevention of herpes simplex infections.

The present invention involves a novel use for natural and synthetic vanilloid compounds. The following are non-limiting examples of such vanilloid compounds, and references in which they are disclosed; capsaicin (trans-8-methyl-N-vanillyl-6-nonenamide) and "synthetic" capsaicin (N-vanillylnonanamide) in U.S. Patent 4,313,958, LaHann, issued February 2, 1982; capsaicin in Yaksh, et al, Science, 206, pp 481-483 (1979); capsaicin in Jancso, et al, Naunyn-Schmiedeberg's Arch. Pharmacol., Vol. 311, pp 285-288 (1980); capsaicin in Holzer et al, Eur. J. Pharm. Vol. 58, pp. 511-514 (1979); 3-hydroxyacetanilide in U.S. Patent 4,238,508, Nelson, issued December 9, 1980; hydroxyphenylacetamides in European Patent Application 0089710, LaHann, et al, published September 28, 1983; N-vanillyl sulfonamides in U.S. Patent 4,401,663, Buckwalter, et al, issued August 30, 1983; hydroxyphenyl-acetamides in U.S. Patent 4,424,205, LaHann, et al, issued January 31, 1984; N-(3- or 4-hydroxy or 3,4-dihydroxybenzyl) carbamates in U.S. Patent 4,443,473, Buckwalter, et al, issued April 17, 1984; N-[(substituted phenyl) methyl]-cis-monounsaturated alkenamides in U.S. Patent 4,493,848, LaHann, et al, issued January 15, 1985; N-(3-methoxy-4-hydroxybenzyl and phenyl) ureas and thioureas in U.S. Patent 4,460,602, Buckwalter, et al, issued July 17, 1984; N-vanillylureas in European Patent Application 0068590, Buckwalter, et al, published January 5, 1983; N-[(substituted phenyl)methyl] alkynamides in U.S. Patent 4,532,139, Janusz, et al, issued July 30, 1985; methylene substituted N-[(substituted phenyl)methyl] alkanamides in U.S. Patent 4,544,668, Janusz, et al, issued October 1, 1985; N-[(substituted phenyl) methyl]-diunsaturated amides in U.S. Patent 4,544,669, LaHann, et al, issued October 1, 1985; monoalkenamides in U.S. Patent 4,564,633, LaHann, et al, issued January 14, 1986; substituted phenylacetic acid esters in British Patent Specification 2,168,974, Loomans, et al, published July 2, 1986; N-(substituted alkyl)alkanamides and thioamides in British Patent Specification 2,168,976, Loomans, et al, published July 2, 1986; substituted aromatic-araalkanamides in British Patent Specification 2,168,975, Janusz et al, published July 2, 1986; and beta-aminoethyl-substituted phenyl compounds in European Patent Application No. 282,127, Gardner, et al., published September 14, 1988.

Vanilloid compounds have been generally disclosed in the above references to have analgesic, anti-irritant and anti-inflammatory activity.

The use of capsaicin to affect the nervous system of humans and other animals, and to treat the pain associated with herpes zoster infections, has been disclosed in a number of references. Handwerker, H.O., U. Holzer-Petsche, Ch. Heym and E. Welk, "C-Fibre Functions After Topical Application of Capsaicin to a Peripheral Nerve and after Neonatal Capsaicin Treatment", Antidromic Vasodilation and Neurogenic Inflammation: Satellite Symposium of the 29th International Congress of Physiological Sciences, Newcastle, Australia, 1983, Edited by Chahl, L.A., J. Szolcsanyi, and F. Lembeck, Akademiai Kiado, Budapest, (1984) pp. 57 - 78, discloses that after repeated capsaicin application, nociceptors become desensitized to subsequent chemical and presumably also heat stimuli. Taylor, D. C. M., Fr.-K. Pierau and J. Szolcsanyi, "The Effect of Capsaicin on Axoplasmic Transport in a Rat Peripheral Nerve", Antidromic Vasodilation and Neurogenic Inflammation: Satellite Symposium of the 29th International Congress of Physioloaical Sciences, Newcastle, Australia, 1983, Edited by Chahl, L.A., Szolcsanyi, J. and F. Lembeck, Akademiai Kiado, Budapest, (1984), pp. 165-171, discloses that systemic capsaicin decreased substance P levels in the dorsal root ganglia and the spinal cord, and that nerve growth factor (NGF) transport in the peripheral nerve may be blocked by systemic capsaicin. Jancso, G., F. Obal, Jr., I. Toth-Kasa, M. Katona and S. Husz, "The Modulation of Cutaneous Inflammatory Reactions by Peptide-Containing Sensory Nerves", International Journal of Tissue Reactions, Vol. VII, (1985), pp. 449-457, discloses a possible role of the nervous system to the mechanisms of inflammation through peptide containing sensory nerves. While rats treated neonatally with capsaicin showed complete abolishment of neurogenic inflammation, repeated topical application of the skin with capsaicin (local desensitization) abolished the neurogenic inflammatory response for several days, along with strongly reduced chemical pain sensitivity and elevated thresholds for warmth and heat pain sensations. Local capsaicin desensitization of the skin prevented whealing, flare and itch in patients with acquired cold and heat urticaria. Jancso, G., S. Husz and N. Simon, "Impairment of Axon Reflex Vasodilatation after Herpes Zoster", Clinical and Experimental Dermatology, Vol. 8, (1983), pp. 27-31, discloses an effect on chemosensitive primary sensory neurons and impairment of axon reflex vasodilatation in skin vessels following herpes zoster infection and the possible mechanisms by which herpes zoster may effect the vascular reactions of the skin.

Capsaicin has been disclosed as an effective compound for the treatment of severe pain associated with herpes zoster infections. Bernstein, J. E., "Capsaicin in the Treatment of Dermatologic Disease", Cutis, Vol. 39, (April 1987), pp. 352-353, discloses use of capsaicin in the possible treatment of post-herpetic neuralgia and psoriasis. Bernstein, J.E., D.R. Bickers, M.V. Dahl, Jay Y. Roshal, "Treatment of Chronic Postherpetic Neuralgia with Topical Capsaicin", Journal of the Americal Academy of Dermatology, Vol. 17, (1981), pp. 93-96, discloses topical application of capsaicin to patients suffering from postherpetic neuralgia as a possible approach for alleviating postherpetic neuralgia and other syndromes characterized by severe localized pain. U.S. Patent 4,536,404, Bernstein, issued August 20, 1985, discloses a method for treating post-herpetic neuralgia due to herpes zoster wherein an effective amount of capsaicin is topically applied to the area affected with herpes zoster to relieve the symptoms of post-herpetic neuralgia.

Capsaicin's effect on the neurogenic response on the skin and in sensory transmission in mice with herpes simplex infections is disclosed in the following two references. Harbour, D. A., T. J. Hill and W. A. Blyth, "Recurrent Herpes Simplex in the Mouse: Inflammation in the Skin and Activation of Virus in the Ganglia Following Peripheral Stimulation", Journal of General Virology, Vol. 64, 1983), pp. 1491-1498, discloses chemical stimuli to the skin which induce recurrent herpes simplex virus disease and reactivation of infectious virus in the ganglia. Capsaicin is injected subcutaneously prior to injection of the mice with dye to help determine the effect of mediators on the permeability of blood vessels. Ljungdahl, A., K. Kristensson, J. M. Lundberg, E. Lycke, B. Svennerholm and R. Ziegler, "Herpes Simplex Virus Infection in Capsaicin-Treated Mice", Journal of the Neurological Sciences, Vol. 72, (1986), pp. 223-230, discloses that capsaicin injected subcutaneously prior to herpes simplex virus (HSV) inoculation of the snouts of four day old (neonatal) mice, reduced the mortality rate of HSV-infected mice.

### Herpes Zoster Infections

Herpes zoster infections are caused by the varicella-zoster virus (VZV), the etiologic agent of the conditions commonly known as shingles, zona and acute posterior ganglionitis.

VZV infections usually cause severe pain and exhibit large groups of lesions distributed along the course of a sensory nerve. Vesicular eruption causes neuralgic pain in the cutaneous area supplied by the peripheral sensory nerves. The vesicular eruptions of herpes zoster are often activated by local lesions involving the posterior root ganglia, systemic diseases such as Hodgkins, and immunosuppressive therapy.

VZV is also the causative agent in chicken pox. Later herpes zoster infections (or shingles outbreaks) are most common after the age of fifty. Crops of vesicles form on an erythemateous base and follow the sensory distribution of one or more posterior root ganglia. The sensory zone on the skin that is affected is usually hyperaesthetic with associated severe pain.

Postherpetic neuralgia is often seen in patients over 60 years of age following a vesicular eruption caused by VZV. Following the eruption, a syndrome of dermatologic rash and pain usually ensues. The rash generally resolves spontaneously in 2 to 3 weeks. The pain may continue and be severe. The treatment of postherpetic neuralgia has often been unsatisfactory and is essentially symptomatic, often requiring potent analgesics and tranquilizers. The postherpetic neuralgia associated with VZV infections may persist for months or years. The pain can be so intractable that it has been implicated in prolonged depression and even suicide. Not infrequently the pain is sufficiently severe and persistent that neurosurgical procedures may be employed in an effort to relieve the patient's discomfort.

Herpes zoster infections rarely recur in a patient (recurrence rate is less than 2%); one attack, generally associated with an outbreak of lesions in any one of numerous areas of the skin surface, usually confers immunity.

### Herpes Simplex Infections

Clinically, herpes zoster infections may have some similarity to herpes simplex virus (HSV) infections, but several important differences between the two exist.

Herpes simplex lesions are characterized by generalized or localized cutaneous and mucosal lesions, often with associated severe constitutional (general, not local) symptoms. Following an acute primary skin infection, the virus spreads along sensory nerves and becomes established in the regional sensory ganglia, or area of neuron cell bodies. HSV usually present with latent infections in the trigeminal or presacrial ganglia. Although VZV infections generally produce latent infections, these occur mainly in the dorsal root ganglia.

HSV infections are generally of two types, Type I or Type II. Type I HSV infections are mainly implicated in oral or ocular herpes infections. HSV Type II infections are usually genital and transmitted primarily through direct contact with herpes lesions. While herpes simplex lesions may appear anywhere on the skin or mucosa, they most frequently appear on the mouth, the lips, the conjunctiva and cornea and the genitalia.

Unlike the 2% recurrence rate of VZV infections, the likelihood of Type I HSV recurrence is 80%, while the likelihood of Type II HSV recurrence is 50%. Reinfection with different strains of Type II HSV may also occur. Recurrent herpetic eruptions can be precipitated by conditions as broad as over exposure to sunlight, febrile illness, physical or emotional stress, or certain foods or drugs.

Postherpetic neuralgia is not generally seen with HSV infections. The primary lesions of HSV (vesicular eruptions) are the most painful, prolonged and widespread. During periods of vesicular eruption, patients often experience pain in the region of viral infection. This pain, though it may be severe, resolves upon healing of the herpetic lesions, and unlike VZV infections, leaves the patient basically asymptomatic between recurrent herpetic episodes. Treatment regimes for recurrent HSV infections have shown little promise in preventing herpes lesions, though there has been some relief of pain and other symptoms.

### Objects of the Present Invention

It is an object of the present invention to provide new methods which are effective for treating or preventing herpes simplex Type I and Type II infections and the attendant recurrent discomfort, lesions and pain.

It is a further object of the present invention to provide medicament for topical treatments to resolve many of the symptoms associated with herpes simplex infections; especially those associated with herpes Type II infections.

It is a still further object of the present invention to provide medicament to be administered topically which produce analgesia without the loss of mechanical sensation (i.e., "numbing") or motor coordination.

It is a still further object of the present invention to provide methods for treating or preventing genital herpes simplex infections and the attendant recurrent discomfort, lesions, and pain (especially herpes simplex Type II infections).

### SUMMARY OF THE INVENTION

The present invention encompasses the use of natural and synthetic vanilloid compounds and the pharmaceutically-acceptable salts thereof for the manufacture of medicaments for treating or preventing herpes simplex infections in humans and lower animals.

This invention particularly relates to to the use of natural and synthetic vanilloid compounds and the pharmaceutically-acceptable salts thereof for the manufacture of medicaments which are topically applied to prevent the recurrence of herpes simplex Type I or Type II lesions.

### DETAILED DESCRIPTION OF THE INVENTION

The term "alkyl", as used herein, means carbon-containing chains which may be straight, branched, or cyclic; substituted or unsubstituted; and which may be saturated, monounsaturated (i.e., one double or triple bond in the chain), or polyunsaturated (e.g., two or more double bonds in the chain; two or more triple bonds in the chain; one or more double and one or more triple bonds in the chain). Unless indicated otherwise, alkyl are preferably as follows. Preferred alkyl are straight or branched chain, especially straight chain. Preferred alkyl are unsubstituted. Preferred alkyl are monounsaturated, especially saturated. Preferred alkyl are C₁-C₂₀, more preferably C₁-C₁₀, more preferably still C₁-C₆, still more preferably C₁-C₄, most preferably C₁-C₂, especially C₁.

The term "carboxylate", as used herein, means an organic carboxylic acid moiety (i.e., -CO₂H), and the salts (e.g., sodium; potassium; calcium; triethylammonium) and esters (e.g., methyl ester; ethyl ester) and amides (e.g., unsubstituted amide; N-methyl amide; N,N-dimethyl amide) thereof which are acceptable from a toxicity viewpoint for administration to humans or lower animals.

The term "aryl", as used herein, means aryl rings which may be mono-, di-, or tri-substituted or unsubstituted (especially substituted or unsubstituted phenyl or napthyl). More preferred aryl is phenyl. More preferred aryl is unsubstituted.

The term "substituted", as used herein for alkyl and aryl groups, means alkyl or aryl groups that can be mono- or polysubstituted. Preferred is mono-, di- or trisubstituted; more preferred is monosubstituted. Preferred substitutents are selected from the group consisting of halogen, hydroxy, amino, thiol, aryl, alkyl, carboxylate and OR wherein R is aryl or an unsubstituted alkyl group (especially methoxy and ethoxy).

As used herein, saturated alkyl groups are referred to as "alkanyl"; unsaturated alkyl groups comprising double bonds in the chain are referred to as "alkenyl" (preferred are chains having the double bonds in the "Z" or "cis" geometric configuration); and unsaturated alkyl groups comprising triple bonds in the chain are referred to as "alkynyl". The designation of geometric configurations for any double bonds present in compounds of the present invention utilizes the art-known nomenclature "Z" and "E", and is fully described in Morrison and Boyd, Organic Chemistry, Third Edition (Allyn and Bacon, Inc., Boston; 1973), pp. 131-133 and 148-151; and March, Advanced Organic Chemistry, Second Edition (McGraw-Hill Book Company, New York; 1977), pp. 86-124; the disclosures of both these references being incorporated herein by reference in their entirety.

The compounds useful in the present invention are natural and synthetic vanilloid compounds, and the pharmaceutically-acceptable salts thereof, having the general structure: 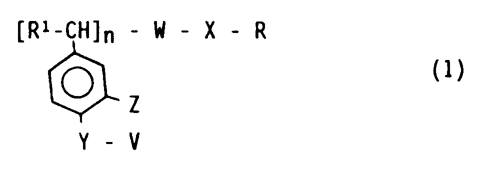
In structure (1) n = 0 or 1.

In structure (1), the -W-X- moiety is selected from -C(O)NH-, -C(S)NH-, -S(O)₂NH-, -NHC(O)O-, -NHC(S)O-, -NHC(O)NH-, -NHC(S)NH-, -C(O)O-, -C(O)S- and -C(O)N(R)-. Preferred -W-X- is selected from -C(O)NH-, -C(S)NH-, -NHC(O)NH-, -NHC(S)NH- and -S(O)₂NH-. More preferred -W-X- is selected from -C(O)NH-, -C(S)NH-, and -NHC(O)NH-. Most preferred -W-X- is -C(O)NH-. Either available bond of the -W-X- moiety may be bonded to the -R moiety, with the other bond being attached to the benzyl carbon atom, or directly attached to the benzene ring.

In structure (1), the -R¹ moiety is selected from hydrogen, hydroxy, alkyl esters of hydroxy having from 1 to 5 carbon atoms, alkyl having from 1 to 5 carbon atoms, and alkoxy having from 1 to 5 carbon atoms. Preferred -R¹ is selected from hydrogen, hydroxy, and methyl; most preferred -R¹ is hydrogen.

In structure (1), the -Z moiety is selected from hydrogen, hydroxy and methoxy; preferred -Z is selected from hydroxy and methoxy. Most preferred -Z is methoxy.

In structure (1), the -Y- moiety is selected from -O-, -S-, -NH-, -OC(O)-, OSO₃²⁻-, and -OPO₃²⁻-; preferred -Y- is selected from -O-, -S- and -NH-. More preferred -Y- is selected from -O-and -S-; most preferred -Y- is -O-.

In structure (1) the -V moiety is selected from hydrogen, -CR²₂-CR²₂-NH₂, and from C₁ to C₃ alkyl. Even more preferred -V is selected from hydrogen and methyl, especially hydrogen. Also more preferred is -CR²₂-CR²₂-NH₂.

The -R² moieties are each independently selected from hydrogen, halogen, unsubstituted or substituted alkyl having from 1 to 5 carbon atoms, substituted or unsubstituted aryl, and carboxylate; or two -R² moieties are covalently bonded to form a substituted or unsubstituted alkyl ring having from 3 to 7 carbon atoms in the ring. It is preferred that at least one -R² on the alpha carbon atom (the carbon atom bonded directly to the Y moiety) be a hydrogen. Preferred also is all -R² being selected from hydrogen and hydroxyalkyl having from 1 to 5 carbon atoms, more preferably 5-hydroxypentyl, 2-hydroxybutyl or hydroxymethyl, especially hydroxymethyl. Preferred also is all -R² being selected from hydrogen and aminoalkyl having from 1 to 5 carbon atoms, more preferably 2-aminopentyl, 2-aminobutyl, aminomethyl or aminoethyl, especially aminomethyl or aminoethyl. Preferred also is all -R² being selected from hydrogen and substituted or unsubstituted aryl, especially phenyl or methylphenyl. Preferred -R² moieties which are aryls include phenyl, naphthyl, and substituted phenyl or naphthyl; most preferred being substituted or unsubstituted phenyl. Preferred -R² moieties which are arylalkyls are substituted, or preferably, unsubstituted. Preferred -R² moieties which are substituted arylalkyls are those where the substituent groups are independently selected from halogen, hydroxy, amino, hydrogen and carboxy groups. Preferred also is all -R² being selected from hydrogen and alkyl having from 1 to 5 carbon atoms (especially methyl). Also preferred is at most only one -R² being other than hydrogen. Also preferred is all -R² being hydrogen.

Particularly preferred is where both -R² on the alpha carbon atom are hydrogen and both -R² on the beta carbon atom (the carbon atom bonded directly to the alpha carbon atom) are unsubstituted or substituted alkyl or are covalently bonded to form a substituted or unsubstituted alkyl or heteroalkyl ring having from 3 to 8 atoms, including from 0 to 3 heteroatoms, in the ring. As used herein, "heteroatoms" means atoms other than carbon that can covalently bond to at least two other atoms and become part of a stable ring structure. Preferred heteroatoms are N, O and S. More preferred -R² on the beta carbon atom are unsubstituted or substituted C₁-C₆ alkyl, more preferably C₁-C₄ alkyl, more preferably still C₁-C₂ alkyl. Also preferred are the two -R² moieties on the beta carbon atom being covalently bonded to form a substituted or unsubstituted alkyl ring having from 3 to 6 carbon atoms, more preferably 3 or 4 or 5 carbon atoms in the ring. Preferred -R² alkyl moieties on the beta carbon atom are saturated or unsaturated having a single double or triple bond, more preferred is that both -R² on the beta carbon be unsubstituted or substituted alkanyl or covalently bonded to form an unsubstituted or substituted alkanyl ring. Preferred substituents of the -R² alkyl moieties on the beta carbon are hydroxy, amino, thiol and carboxylate, especially hydroxy and amino. More preferred is that all -R² alkyl moieties on the beta carbon being unsubstituted. More preferred still is that both -R² on the beta carbon atom are methyl or ethyl, especially methyl.

In structure (1) the -R moiety is a C₂-C₂₄ alkyl moiety which may be straight, branched or cyclic chain and may be saturated, monounsaturated, or polyunsaturated, substituted or unsubstituted. Preferred -R moieties are straight and branched chain alkanyl, straight and branched chain monounsaturated alkyl, straight and branched chain diunsaturated alkyl, and straight and branched chain triunsaturated alkyl. More preferred -R moieties are mono or diunsaturated or saturated, C₆-C₂₄ straight or branched chain alkyls. Also more preferred are C₅-C₁₁ straight chain alkyls, especially C₇-C₁₀ straight chain alkanyls. Even more preferred are mono or diunsaturated alkenyls, or C₆-C₂₄ straight chain alkenyls. Further preferred are monounsaturated cis-double bond C₁₁-C₂₃ straight chain alkenyls. Even further preferred is mono-unsaturated cis-double bond C₁₇-C₂₃ straight chain alkenyls. Most preferred -R is 9-Z-octadecenyl.

The preferred -R groups are as follows. For the medicaments to be used according to the present invention which comprise phenylacetic acid amide or thioamide derivatives, particularly the beta-aminoethoxy-substituted compounds have the general structure: 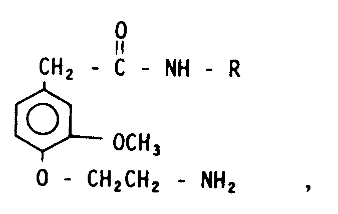
the preferred -R groups are selected from n-hexanyl, n-heptanyl, n-octanyl, n-nonanyl, n-decanyl, n-undecanyl, n-dodecanyl, n-tridecanyl, n-tetradecanyl, tetradecenyl, pentadecenyl, hexadecenyl, heptadecenyl, octadecenyl, nonadecenyl, eicosenyl, docosenyl, octadecadienyl, nonadecadienyl, eicosadienyl, octadecatrienyl, eicosatrienyl, eicosatetraenyl, octadecynyl, nonadecynyl, eicosynyl, and docosynyl. More preferred -R groups are selected from n-octanyl; n-nonanyl; n-decanyl; 9E- or 9Z-tetradecenyl; 9E- or 9Z-hexadecenyl; 9E- or 9Z-octadecenyl; 6E- or 6Z-octadecenyl; 11E-or 11Z-octadecenyl; 10E- or 10Z-nonadecenyl; 13E- or 13Z-docosenyl; 9-methylene-1-octadecanyl, 9Z; 12Z-octadecadienyl; 9E, 12E-octadecadienyl; 9Z, 12E-octadecadienyl; 9Z, 11E-octadecadienyl; 10E, 13E-nonadecadienyl; 11E, 14E-eicosadienyl; 9Z, 12Z, 15Z-octadecatrienyl; 6Z, 9Z, 12Z-octadecatrienyl; 11Z, 14Z, 17Z-eicosatrienyl; 5Z, 8Z, 11Z, 14Z-eicosatetraenyl; and 9-octadecynyl. Most preferred -R groups are n-octanyl, n-nonanyl, and 9Z-octadecenyl.

For the compounds of the present invention which are vanillylamide or vanillylthioamide derivatives, particularly the beta-aminoethoxy-substituted compounds having the general structure: 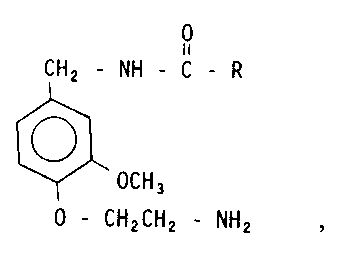
the preferred -R groups are selected from n-hexanyl, n-heptanyl, n-octanyl, n-nonanyl, n-decanyl, n-undecanyl, n-dodecanyl, n-tridecanyl, tridecenyl, tetradecenyl, pentadecenyl, hexadecenyl, heptadecenyl, octadecenyl, nonadecenyl, eicosenyl, docosenyl, heptadecadienyl, octadecadienyl, nonadecadienyl, eicosadienyl, heptadecatrienyl, octadecatrienyl, nonadecatrienyl, eicosatrienyl, nonadecatetraenyl, heptadecynyl, octadecynyl, nonadecynyl, and eicosynyl. More preferred -R groups are selected from n-heptanyl; n-octanyl; n-nonanyl; 8E- or 8Z-tridecenyl; 8E- or 8Z-pentadecenyl; 8E- or 8Z-heptadecenyl; 5E- or 5Z-heptadecenyl; 10E- or 10Z-heptadecenyl; 9E- or 9Z-octadecenyl; 12E- or 12Z-nonadecenyl; 8-methylene-1-heptadecanyl; 8Z, 11Z-heptadecadienyl; 8E, 11E-heptadecadienyl; 8Z, 11E-heptadecadienyl; 8Z, 10E-heptadecadienyl; 9E, 12E-octadecadienyl; 10E, 13E-nonadecadienyl; 8Z, 11Z, 14Z-heptadecatrienyl; 5Z, 8Z, 11Z-heptadecatrienyl; 10Z, 13Z, 16Z-nonadecatrienyl; 4Z, 7Z, 10Z, 13Z-nonadecatetraenyl; and 8-heptadecynyl. Most preferred -R groups are n-heptanyl, n-octanyl and 8Z-heptadecenyl (i.e., oleoyl amide).

The -R alkyl groups may be substituted or, preferably, unsubstituted. Preferred substituents are selected from the group consisting of halogen, hydroxy, amino, aryl, carboxylate, and -OR³ wherein -R³ is an unsubstituted alkyl group having from 1 to 3 carbon atoms (especially methoxy and ethoxy). It is preferred that substituted alkyl groups be mono-, di- or trisubstituted, most preferably monosubstituted. When -W-X- is -C(O)N(R)-, the two -R groups may be covalently bonded to form a substituted or unsubstituted alkyl ring having from 2 to 14 carbon atoms in the ring.

The term "pharmaceutically-acceptable salts and amides", as used herein, means the compounds in their salt or amide form which have the same general pharmacological properties as the basic amino form from which they are derived, and which are acceptable from a toxicity viewpoint. Pharmaceutically-acceptable salts include ammonium salts derived from inorganic acids (e.g., HCl, HBr, NaHSO₄, H₂CO₃), and ammonium carboxylic acid salts derived from organic carboxylic acids (e.g., acetic acid; lactic acid; gluconic acid; citric acid; glucuronic acid; galacturonic acid; fumaric acid; gentisic acid; lactobionic acid; benzoic acid). Pharmaceutically-acceptable amides include those derived from organic carboxylic acids (e.g., acetic acid amides) including amino acids (e.g., glycine amides). Preferred are the ammonium carboxylic acid salts derived from organic carboxylic acids, especially the acetate and lactate salts.

Preferred compounds suitable in the medicaments to be used according to the present invention include 8-methyl-N-vanillyl-6-nonenamide; N-vanillylnonanamide; N-vanillyl-9-octadecenamide; N-((4-(2-aminoethoxy)-3-methoxyphenyl)-methyl)-9Z-octadecenamide; N-((4-(2-aminoethoxy)-3-methoxyphenyl)-methyl)-nonanamide; N-((4-(2-methyl-2-aminopropoxy)-3-methoxyphenyl)-methyl)-nonanamide; N-((4-(2-methyl-2-aminopropoxy)-3-methoxyphenyl)-methyl)-9Z-octadecenamide; N-((4-(2-amino-3-methylbutoxy)-3-methoxyphenyl)-methyl)-9Z-octadecenamide; N-((4-(1-amino-1-cyclopropylmethoxy)-3-methoxyphenyl)-methyl)-9Z-octadecenamide; N-((4-(1-amino-1-cyclopentylmethoxy)-3-methoxy)phenyl)methyl)-9Z-octadecenamide; N-(9Z-octadecenyl)-4-(2-amino-2-methylpropoxy)-3-methoxyphenylacetamide; N-(9Z-octadecenyl)-4-(2-aminoethoxy)-3-methoxyphenylacetamide; N-octanyl-4-(2-aminoethoxy)-3-methoxyphenylacetamide; N-((4-(2-amino-3-hydroxypropoxy)-3-methoxyphenyl)-methyl)-9Z-octadecenamide; N-((4-(2-amino-2-carboxyethoxy)-3-methoxyphenyl)-methyl)-9Z-octadecenamide; and the pharmaceutically-acceptable salts and amides thereof. More preferred compounds suitable in the medicaments to be used according to the present invention include 8-methyl-N-vanillyl-6-nonenamide; N-vanillylnonanamide; N-vanillyl-9-octadecenamide; N-((4-(2-aminoethoxy)-3-methoxyphenyl)-methyl)-9Z-octadecenamide; N-(9Z-octadecenyl)-4-(2-aminoethoxy)-3-methoxyphenylacetamide; N-((4-(2-aminoethoxy)-3-methoxyphenyl)-methyl)-nonanamide; N-((4-(2-methyl-2-aminopropoxy)-3-methoxyphenyl)-methyl)-nonanamide; N-((4-(2-methyl-2-aminopropoxy)-3-methoxyphenyl)-methyl)-9Z-octadecenamide; N-(9Z-octadecenyl)-4-(2-amino-2-methylpropoxy)-3-methoxyphenylacetamide and the pharmaceutically-acceptable salts and amides thereof.

As noted hereinbefore, capsaicin and a wide variety of other substituted phenyl compounds are known to have analgesic activity. Heretofore, however, the capsaicinoids and other vanilloid compounds have not been shown to have an effect on herpes simplex infections. Surprisingly, natural and synthetic vanilloid compounds of the present invention are efficacious to help prevent and/or treat recurrent herpes simplex infections.

Specific pharmaceutical compositions useful in this invention are described in the following U.S. Patents , U.S. Patent No. 4,401,663, Buckwalter, et al, issued August 30, 1983; U.S. Patent No. 4,424,205, LaHann, et al, issued January 31, 1984; U.S. Patent No. 4,443,473, Buckwalter, et al, issued April 12, 1984; U.S. Patent No. 4,493,848, LaHann, et al, issued January 15, 1985. Representative pharmaceutical compositions useful in the methods of the present invention are provided in the non-limiting Examples provided hereinafter. Such pharmaceutical compositions preferably comprise one or more of the vanilloid compounds and a pharmaceutically acceptable carrier.

The term "pharmaceutically-acceptable carrier", as used herein, means one or more compatible solid or liquid filler diluents or encapsulating substances which are suitable for administration to a human or lower animal. The term "compatible", as used herein, means that the components of a pharmaceutical carrier are capable of being commingled with the vanilloid compounds and with each other, in a manner such that there is no interaction which would substantially reduce the pharmaceutical efficacy of the pharmaceutical composition under ordinary use situations. Pharmaceutically-acceptable carriers must, of course, be of sufficiently high purity and sufficiently low toxicity to render them suitable for administration to the human or lower animal being treated.

The pharmaceutically-acceptable carrier employed in the medicaments to be used according to the present invention is used at a concentration sufficient to provide a practical size to dosage relationship. The pharmaceutically-acceptable carriers, in total, may comprise from 50% to 99.99% by weight of the pharmaceutical compositions of the present invention, preferably from 90% to 99.9%, and more preferably from 95% to 99.5%.

Total single dosages of the vanilloid compounds present in pharmaceutical compositions useful herein are generally from 1 ug to 1 g. Preferred single dosages are from 10 ug to 100 mg; more preferred are from 100 ug to 50 mg; and most preferred are from 1 mg to 10 mg.

The choice of a pharmaceutically-acceptable carrier to be used in conjunction with the compounds of the present invention is largely determined by the way the compound is to be administered. Such methods include parenteral (especially subcutaneous), oral and topical. As used herein, "administered topically" means placing the compounds in contact with the skin, mucous membrane, or body cavity. It thus includes epidermal, intraoral, intranasal, intravaginal, intraanal, intrauretheral, intraaural, and extraocular administration. The preferred use of the vanilloid compounds for the manufacture of a medicament according to the present invention is topical administration of said compounds. Suitable pharmaceutically-acceptable carriers for topical application include those suited for use in lotions, creams, solutions, gels, tapes and the like.

Suitable carriers for topical administration preferably remain in place on the skin as a continuous film and resist being washed off easily by perspiration or by immersion in water. Generally, the carrier is either organic in nature or an aqueous emulsion and capable of having the vanilloid compound dispersed or dissolved therein. The carrier may include pharmaceutically-acceptable emollients, skin penetration enhancers, coloring agents, fragrances, emulsifiers, thickening agents, and solvents. A more detailed description of such forms follows:

### 1. Lotions

The lotions can comprise an effective amount (preferably from 0.001% to 5%, more preferably from 0.1% to 1%) of a vanilloid compound; from 1% to 50%, preferably from 3% to 15%, of an emollient; the balance being water, a C₂ or C₃ alcohol, or a mixture of water and the alcohol. Several emollients are known. Examples of such emollients are as follows:
a. Hydrocarbon oils and waxes. Examples are mineral oil, petrolatum, paraffin, ceresin, ozokerite, microcrystalline wax, polyethylene, and perhydrosqualene.
b. Silicone oils, such as dimethylpolysiloxanes, methylphenylpolysiloxanes, water-soluble and alcohol-soluble silcone-glycol copolymers.
c. Triglyceride fats and oils such as those derived from vegetable, animal and marine sources. Examples include castor oil, safflower oil, cotton seed oil, corn oil, olive oil, cod liver oil, almond oil, avocado oil, palm oil, sesame oil, and soybean oil.
d. Acetoglyceride esters, such as acetylated monoglycerides.
e. Ethoxylated glycerides, such as ethoxylated glyceryl monostearate.
f. Alkyl esters of fatty acids having 10 to 20 carbon atoms. Methyl, isopropyl and butyl esters of fatty acids are useful herein. Examples include hexyl laurate, isohexyl laurate, isohexyl palmitate, isopropyl palmitate, isopropyl myristate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
g. Alkenyl esters of fatty acids having 10 to 20 carbon atoms. Examples thereof include oleyl myristate, oleyl stearate, and oleyl oleate.
h. Fatty acids having 9 to 22 carbon atoms. Suitable examples include pelargonic, lauric, myristic, palmitic, stearic, isostearic, hydroxystearic, oleic, linoleic, ricinoleic, arachidonic, behenic, and erucic acids.
i. Fatty alcohols having 10 to 22 carbon atoms. Lauryl, myristyl, cetyl, hexadecyl, stearyl, isostearyl, hydroxystearyl, oleyl, ricinoleyl, behenyl, erucyl, and 2-octyl dodecyl alcohols are examples of satisfactory fatty alcohols.
j. Fatty alcohol ethers. Ethoxylated fatty alcohols of 10 to 20 carbon atoms include the lauryl, cetyl, stearyl, isostearyl, oleyl, and cholesterol alcohols having attached thereto from 1 to 50 ethylene oxide groups or 1 to 50 propylene oxide groups, or a mixture thereof.
k. Ether-esters such as fatty acid esters of ethoxylated fatty alcohols.
l. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols, lanolin alcohols linoleate, lanolin alcohols ricinoleate, acetate of lanolin alcohols ricinoleate, acetate of ethoxylated alcohols-esters, hydrogenolysis or lanolin, ethoxylated hydrogenated lanolin, ethoxylated sorbitol lanolin, and liquid and semisolid lanolin absorption bases are illustrative of emollients derived from lanolin.
m. Polyhydric alcohols and polyether derivatives. Propylene glycol, dipropylene glycol, polypropylene glycol (M.W. 2000-4000), polyoxyethylene polyoxypropylene glycols, polyoxypropylene polyoxyethylene glycols, glycerol, ethoxylated glycerol, propoxylated glycerol, sorbitol, ethoxylated sorbitol, hydroxypropyl sorbitol, polyethylene glycol (M.W. 200-6000), methoxy polyethylene glycols 350, 550,750, 2000, 5000 poly[ethylene oxide] homopolymers (M.W. 100,000-5,000,000), polyalkylene glycols and derivatives, hexylene glycol (2-methyl-2,4-pentanediol), 1,3-butylene glycol, 1,2,6-hexanetriol, ethohexadiol USP (2-ethyl-1,3-hexanediol) C₁₅-C₁₈ vicinal glycol, and polyoxypropylene derivates of trimethylolpropane are examples thereof.
n. Polyhydric alcohol esters. Ethylene glycol mono- and di-fatty acid esters, diethylene glycol mono- and di-fatty acid esters, polyethylene glycol (M.W. 200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxy-ethylene sorbitan fatty acid esters are satisfactory polyhydric alcohol esters.
o. Wax esters such as beeswax, spermaceti, myristyl myristate, stearyl stearate.
p. Beeswax derivatives, e.g., polyoxyethylene sorbitol beeswax. These are reaction products of beeswax with ethoxylated sorbitol or varying ethylene oxide content, forming a mixture of ether-esters.
q. Vegetable waxes including carnauba and candelilla waxes.
r. Phospholipids such as lecithin and derivatives.
s. Sterols. Cholesterol, cholesterol fatty acid esters are examples thereof.
t. Amides such as fatty acid amides, ethoxylated fatty acid amides, solid fatty acid alkanolamides.

The lotions further preferably comprise from 1% to 10%, more preferably from 2% to 5%, of an emulsifier. The emulsifiers can be nonionic, anionic or cationic. Examples of satisfactory nonionic emulsifiers include fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono- and di-fatty acid esters of ethylene oxide, mono- and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters. Suitable anionic emulsifiers include the fatty acid soaps, e.g. sodium, potassium and triethanolamine soaps, wherein the fatty acid moiety contains from 10 to 20 carbon atoms. Other suitable anionic emulsifiers include the alkali metal, ammonium or substituted ammonium alkyl sulfates, alkyl arylsulfonates, an alkyl ethoxy ether sulfonates having 10 to 30 carbon atoms in the alkyl moiety. The alkyl ethoxy ether sulfonates contain from 1 to 50 ethylene oxide units. Satisfactory cationic emulsifiers are the quaternary ammonium, morpholinium and pyridinium compounds. Certain of the emollients described in preceding paragraphs also have emulsifying properties. When a lotion is formulated containing such an emollient, an additional emulsifier is not needed, though it can be included in the composition.

The balance of the lotion is water or a C₂ or C₃ alcohol, or a mixture of water and the alcohol. The lotions are formulated by simply admixing all of the components together. Preferably the compound of the present invention is dissolved in the mixture. Conventional optional components can be included. One such additive is a thickening agent at a level from 1% to 10% of the composition. Examples of suitable thickening agents include: cross-linked carboxypolymethylene polymers, ethyl cellulose, polyethylene glycols, gum tragacanth, gum kharaya, xanthan gums and bentonite, hydroxyethyl cellulose, and hydroxypropyl cellulose.

### 2. Creams

The creams comprise an effective amount (preferably from 0.001% to 5%, more preferably from 0.1% to 1%) of a vanilloid compound; from 5% to 50%, preferably from 10% to 25%, of an emollient; the balance being water. The emollients above described can also be used in the cream compositions. Optionally the cream form contains a suitable emulsifier, as previously described. When an emulsifier is included, it is in the composition at a level from 3% to 50%, preferably from 5% to 20%.

### 3. Solutions

The solution form comprises an effective amount (preferably from 0.001% to 5%, more preferably from 0.1% to 1%) of a vanilloid compound; the balance being water and/or a suitable organic solvent. Suitable organic materials useful as the solvent or a part of a solvent system are as follows: propylene glycol, polyethylene glycol (M.W. 200-600), polypropylene glycol (M.W. 425-2025), glycerine, sorbitol esters, 1,2,6-hexanetriol, ethanol, isopropanol, diethyl tartrate, butanediol, and mixtures thereof. Such solvent systems can also contain water.

These compositions in solution form can be applied to the skin as is, or else can be formulated into an aerosol and applied to the skin as a spray-on. The aerosol compositions further comprise from 25% to 80%, preferably from 30% to 50%, of a suitable propellant. Examples of such propellants are the chlorinated, fluorinated and chlorofluorinated lower molecular weight hydrocarbons. Nitrous oxide, carbon dioxide, butane, and propane are also used as propellant gases. These propellants are used at a level sufficient to expel the contents of the container.

### 4. Gels

Gel compositions can be formulated by simply admixing a suitable thickening agent to the previously described solution compositions. Examples of suitable thickening agents have been previously described with respect to the lotions.

The gelled compositions comprise an effective amount (preferably from 0.001% to 5%, more preferably from 0.1% to 1%) of a vanilloid compound; from 5% to 75%, preferably from 10% to 50%, of an organic solvent as previously described; from 0.5% to 20%, preferably from 1% to 10% of the thickening agent; the balance being water.

### 5. Solids

Compositions of solid forms have use as stick-type compositions intended for application to the lips or other parts of the body. Such compositions comprise an effective amount (preferably from 0.001% to 5%, more preferably from 0.1% to 1%) of a vanilloid compound, and from 50% to 98%, preferably from 60% to 90%, of the previously described emollients. This composition can further comprise from 1% to 20%, preferably from 5% to 15%, of a suitable thickening agent, and optionally emulsifiers and water. Thickening agents previously described with respect to lotions are suitable herein.

### 6. Genital Formulations

Compositions used in the present invention can be administered in a wide variety of vehicles, especially in the genital region. In addition to general skin treatment, infections in the genital area can be treated using vaginal, anal or uretheral suppositories; vaginal pessaries; vaginal or rectal tablets or inserts; catamenial and non-catamenial tampons; ointments; enemas; cones; emulsions; and douches.

Additives commonly found in topical compositions such as preservatives, e.g., methyl and ethyl-paraben, dyes and perfume can be included in any of the previously described topical compositions.

The present invention involves the use of the medicaments according to the present invention for treating herpes simplex infections, alleviating signs and symptoms associated with herpetic vesicular eruptions and the attendant pain of herpes simplex lesion. The use of the medicaments according to the present invention may be particularly adapted for preventing recurrent herpes simplex infections and/or for relieving the symptoms associated with herpes simplex vesicular eruptions.

The phrase "safe and effective amount", as used herein, means an amount of a compound or composition high enough to significantly positively modify the condition to be treated, but low enough to avoid serious side effects (at a reasonable benefit/risk ratio), within the scope of sound medical judgment. The safe and effective amount of the compound or composition will vary with the particular condition being treated, the age and physical condition of the patient being treated, the severity of the condition, the duration of the treatment, the nature of concurrent therapy, the specific compound or composition employed, the particular pharmaceutically-acceptable carrier utilized, and like factors within the knowledge and expertise of the attending physician. Daily dosages can range from 0.01 mg/kg of body weight to 5 mg/kg of body weight. Preferred daily dosages are from 0.1 to 2 mg/kg of body weight. Up to 6 single dosages per day may be administered.

Topical administration can be used by directly laying on or spreading a safe and effective amount of a vanilloid composition on epidermal or epithelial tissue, including outer skin and oral, anal, vaginal, gingival, and nasal tissue. The amount of the pharmaceutical composition to be topically administered may vary from 1 mg/cm² to 20 mg/cm², and if a patch is worn over the affected area possibly higher amounts, depending upon such factors as the sensitivity, type and location of tissue to be treated, the composition and carrier to be administered, and the particular compound to be administered.

An animal model which can be used to demonstrate the use of vanilloids for the prevention or treatment of herpes simplex infections is disclosed in Stanberry, L.R., R. L. Burke and M. G. Myers, "Herpes Simplex Virus Glycoprotein Treatment of Recurrent Genital Herpes", The Journal of Infectious Diseases, Vol. 157, (1988), pp. 156-163; and Stanberry, L. R., S. Kit and M. G. Myers, "Thymidine Kinase-deficient Herpes Simplex Virus Type 2 Genital Infection in Guinea Pigs", Journal of Virology, Vol. 55, (1985), pp. 322-328.

### Example I

Female Hartley guinea pigs (300-350g) are randomized into groups of 12. A mixture of Tween®, EtOH and 0.9% NaCl in distilled water is made to obtain the following concentrations (v/v):
10% Tween® 80
10% EtOH (ethanol)
80% 0.9%NaCl in distilled H₂O
The ethanol/Tween®/saline (ETS) vehicle is mixed with capsaicin to obtain a concentration of capsaicin of 1.0%. Capsaicin is administered intravaginally in a 100 microliter volume of the (ETS) vehicle.

The guinea pigs are intravaginally inoculated with 5.0 log pfu HSV-II, MS strain, following capsaicin administration. The virologic course of primary infection 1-10 days post inoculation is assessed by determining the titer of virus in secretions collected by swabbing the vagina daily. The clinical course of primary infection is assessed, along with the incidence and duration of urinary retention, hindlimb paralysis, and the incidence of death occurring within 14 days of viral challenge. The frequency and severity of recurrent disease is determined by daily examination of the guinea pig after recovery from the primary infection of days 15-21 through day 100. Animals treated with capsaicin show zero or few lesions, whereas the untreated animals show numerous lesions. Similar results are obtained using a 1.0% concentration of N-vanillylnonanamide in the ETS vehicle.

Similar results are also obtained using a 1.0% concentration of N-((4-2-aminoethoxy)-3-methoxyphenyl)-methyl)-9Z-octadeccenamide in a vehicle consisting of 10% EtOH, 10% Tween® 80, and 80% of an aqueous buffer solution consisting of acetic acid (6 g/l) and sodium hydroxide (2 g/l) in distilled water.

### Example II

### Synthesis of N-((4-(2-aminoethoxy)-3-methoxyphenyl)-methyl) -9Z-octadecenamide by the Phthalimide Synthesis Method

### (a) Synthesis of N-((4-(2-bromoethoxy)-3-methoxyphenyl)methyl)-9Z-octadecenamide:

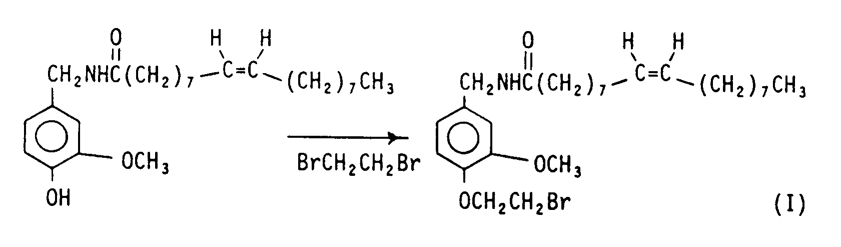

N-vanillyloleamide (100 gm, 0.24 mole; which is prepared as described in U.S. Patent 4,493,848 to LaHann and Buckwalter, issued January 15, 1985), 1,2-dibromoethane (500 ml), 40% KOH (165 ml) and 40% tetrabutylammonium hydroxide (15 ml) are combined in a 2 L flask equipped with a thermometer, mechanical stirrer and reflux condenser, and the solution is heated to 55°C. The disappearance of starting material is monitored by TLC (6% acetone/CH₂Cl₂). After the reaction is completed (typically after reacting overnight), the solution is diluted with CHCl₃ (500 ml), washed three times with water and the aqueous washings are then combined and extracted once with CHCl₃ (250 ml). The organic phases are combined, washed with 10% H₃PO₄ (250 ml), saturated NaHCO₃ (250 ml), brine, then dried (MgSO₄) and filtered. The volume is reduced to less than 500 ml by vacuum distillation and the liquid is then transferred to a 4-L flask. After cooling to approximately 10°C, Et₂O (2L) is added and the flask is cooled in and ice bath. A white precipitate forms and additional Et₂O is added to make a volume of 4 l. The thick amorphous solid is filtered, washed with Et₂O, dried and used without further purification. Mp=96-97°C. ¹H NMR (CDCl₃) (ppm): 6.8 (s, 3H), 5.9 (m, 1H), 5.3 (t, 2H J=5Hz), 4.3 (m, 4H), 3.8 (s, 3H), 3.6 (t, 2H, J=6.5 Hz), 2.3-2.1 (m, 4H), 2.0-1.5 (m, 2H), 1.2 (s, 22H), 0.85 (t, 3H). ¹³C NMR (CDCl₃) (ppm): 14.0, 22.5, 25.6, 27.0, 29.1, 31.7, 36.5, 43.0, 55.8, 69.3, 111.9, 115.0, 119.8, 129.5, 132.8, 146.5, 149.8, 172.8.

### (b) Synthesis of N-((4-(2-phthalimidoethoxy)-3-methoxy-phenyl)-methyl)-9Z-octadecenamide:

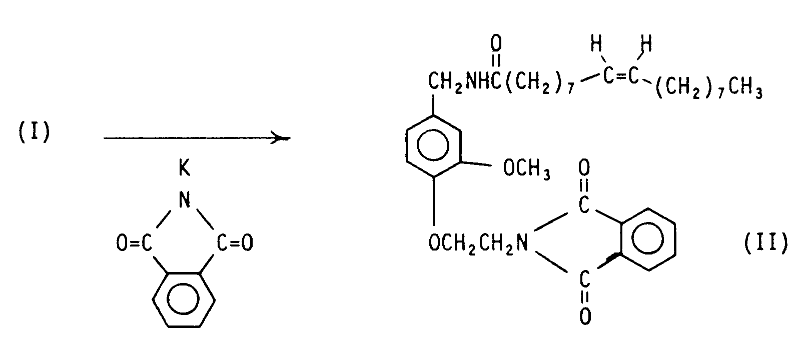

N-((4-(2-bromoethoxy)-3-methoxyphenyl)-methyl)-9Z-octadecenamide (615.5 gm, 1.17 moles) and DMF are combined in a 5 L flask equipped with a mechanical stirrer and a thermometer. The mixture is stirred and heated to 55°C. Once the solution becomes homogeneous, potassium phthalimide (270.0 gm, 1.47 mole) is added and the solution is maintained at 55°C with good stirring. After ca. 10 minutes the reaction solution becomes homogeneous. Precipitation of a white solid then begins and the reaction is allowed to stir overnight at 55°C. TLC (acetone/dichloromethane 6:94) indicates when the starting material has been consumed. The reaction solution is divided into three equal volumes and each is worked up as follows: The solution is poured into a 4 L beaker and then diluted with water to the 3500 mL mark. After standing for ca. 15 minutes the solution is divided again into two equal parts, each of which is diluted to 3500 mL with water. The white precipitate is removed by filtration and washed several times with water. The three portions are combined and dried in a vacuum oven at 90°C for 12 hours to give 682.25 g (98%) of a fine powder. Mp 109.5 - 111.0°C. ¹H NMR (CDCl₃)(ppm): 7.8 (m, 4H), 6.8 (m, 3H), 5.8 (m, 1H), 5.3 (t, 2H), 4.3 (d, 2H), 4.2 (t, 2H), 4.1 (t, 2H), 3.7 (s, 3H), 2.2 - 2.0 (m, 4H), 1.6 (m, 2H), 1.2 (s, 22H), 0.9 (t, 3H), ¹³C NMR (CDCl₃)(ppm): 172.8, 167.9, 150.0, 147.1, 133.9, 132.5, 132.1, 129.7, 123.1, 120.0, 114.9, 112.0, 66.2, 55.7, 43.2, 37.1, 36.7, 31.8, 29.3, 27.1, 25.7, 22.6, 14.0. IR (cm-¹): 3300, 1775, 1715, 1635, 1265, 1230, 1145, 1035, 1025, 720, CI-DEP Mass spectrum (m/z): 591 (MH+).

### (c) Synthesis of N-((4-(2-aminoethoxy)-3-methoxyphenyl)-methyl)-9Z-octadecenamide:

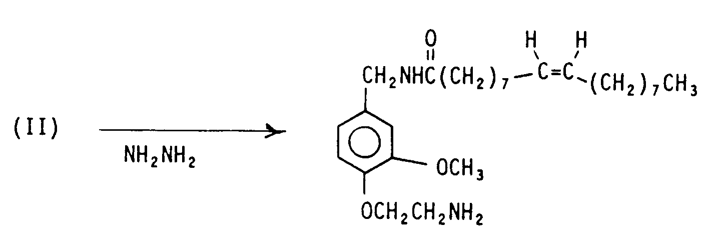

N-((4-(2-phthalimidoethoxy)-3-methoxyphenyl-methyl)-9Z-octadecenamide (250 gm, 0.424 mole) and ethanol (2500 mL) are combined in a 4 L beaker. The slurry is mechanically stirred and heated to 60°C. At ca. 45°C the solution becomes homogeneous. 1-Hexene (20 mL) is added. Hydrazine hydrate (106 mL of a 64% aqueous solution) is then added. In ca. 5 minutes a white precipitate begins to form. During the 2 hour reaction time 500 mL of ethanol is added to the reaction to replenish that volume lost to evaporation. The reaction solution is then divided into three equal portions and each is worked up as follows: Methyl t-butyl ether (1.5 L) is used to transfer the slurry into a 4 L separatory funnel. Water (1 L) and 1N NaOH (500 mL) are added and the solution is thoroughly shaken. 50% NaOH (25 mL) is added and the solution is reshaken. The organic phase is then extracted twice with alkali using the same sequence, and washed with brine. The extract is dried over sodium sulfate and concentrated. The crude product is taken up in hot methyl t-butyl ether and allowed to crystallize. The crystals are filtered and dried in a vacuum desicator to give 124.87 g. A second crop of crystals, 40.82 g, is obtained to give 85% total yield. Mp 102-106°C. ¹H NMR CDCl₃)(ppm): 6.7 (s, 3H), 6.1 (m, 1H), 5.3 (t, 2H), 4.3 (d, 2H), 3.9 (t, 3H), 3.8 (s, 3H), 3.0 (t, 2H), 2.5 (s, 2H), 2.1-1.6 (m, 4H), 1.5-1.2 (m, 2H), 1.2 (s, 22H), 0.9 (t, 3H). ¹³C NMR (CDCl₃) (ppm): 172.9, 149.3, 147.2, 131.8, 129.6, 119.7, 113.5, 111.4, 70.9, 55.5, 42.9, 40.9, 36.4, 31.6, 29.0, 26.9, 25.6, 22.4, 13.8. IR max (cm-¹): 3380, 3300, 1630, 1375, 1255, 1235, 1020, 800, 720 cm-1. CI-DEP Mass Spectrum (m/z): 461 (MH+).

## Claims

1. The use of a compound for the manufacture of a medicament to be administered topically for treating or preventing vesicular eruptions (lesions) due to herpes simplex infections in a human or lower animal characterized in that the compound has the general structure: 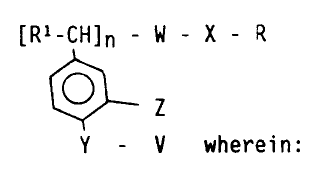
a) n is 0 or 1, preferably 1;
b) -W-X- is selected from -C(O)NH-, -C(S)NH-, -S(O)₂NH-, -NHC(O)O-, -NHC(S)O-, -NHC(O)NH-, -NHC(S)NH-, -C(O)O-, -C(O)S-, and -C(O)N(R)-; preferably from -C(O)NH-, -C(S)NH-, -NHC(O)NH-, -NHC(S)NH-, and -S(O)₂NH-; more preferably from -C(O)NH- and -C(S)NH-; where either available bond of -W-X- is bonded to -R and the other bond is attached to the benzyl carbon atom or to the benzene ring;
c) -R¹ is selected from hydrogen, hydroxy, alkyl esters of hydroxy having from 1 to 5 carbon atoms, alkyl having from 1 to 5 carbon atoms, and alkoxy having from 1 to 5 carbon atoms; preferably from -H, -OH and -CH₃; more preferably -H;
d) -Z is selected from -H, -OH, -OCH₃; preferably from -OH and -OCH₃; more preferably -OCH₃;
e) -Y- is selected from -O-, -S-, -NH-, -OC(O)-, -OSO₃²⁻-, and -OPO₃²⁻-, preferably from -O-, -S- and -NH-; more preferably -O-;
f) -V is selected from -H, from C₁ to C₃ alkyl, and -CR²₂-CR²₂-NH₂;
g) -R² moieties are independently selected from hydrogen, halogen, unsubstituted or substituted alkyl having from 1 to 6 carbon atoms, substituted or unsubstituted aryl, and carboxylate, or two -R² moieties are covalently bonded to form a substituted or unsubstituted alkyl or heteroalkyl ring having from 3 to 8 atoms in the ring including from 0 to 3 heteroatoms; and
h) -R is selected from C₂-C₂₄ alkyl which is straight, branched, or cyclic chain; saturated, mono-unsaturated or polyunsaturated; and substituted or unsubstituted; or two -R moieties are covalently bonded to form a substituted or unsubstituted alkyl ring having from 2 to 14 carbon atoms in the ring; preferably -R is selected from unsubstituted, saturated or mono- or di-unsaturated, C₆-C₂₄ straight or branched chain alkyl;
or the pharmaceutically-acceptable salts and amides thereof.

2. The use of Claim 1 characterized in that all -R² on the alpha carbon are -H; both -R² on the beta carbon are selected from -H, unsubstituted or substituted C₁-C₆ alkyl or the -R² are covalently bonded to form a substituted or unsubstituted C₃-C₆ ring, preferably both -R² on the beta carbon are selected from methyl and ethyl or are bonded to form cyclopropyl, cyclobutyl or cyclopentyl, more preferably both are methyl; and -R is saturated or mono- or di-unsaturated with double bonds, C₆-C₂₄ straight or branched chain alkyl; preferably mono-unsaturated, cis-double bond, C₁₁-C₂₃ straight chain alkenyl or C₇-C₁₀ straight chain alkanyl.

3. The use of Claim 2 characterized in that -V is selected from -H, and -CH₃.

4. The use of Claim 3 characterized in that said compound is trans-8-methyl-N-vanillyl-6-nonenamide or N-vanillylnonanamide.

5. The use of Claim 2, characterized in that -V is -CR²₂-CR²₂-NH₂.

6. The use of Claim 5 characterized in that the compound has the general structure: 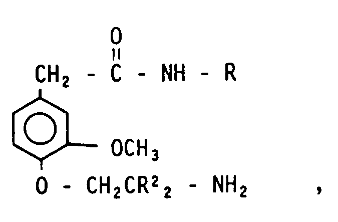 wherein -R is selected from n-hexanyl, n-heptanyl, n-octanyl, n-nonanyl, n-decanyl, n-undecanyl, n-dodecanyl, n-tridecanyl, n-tetradecanyl, tetradecenyl, pentadecenyl, hexadecenyl, heptadecenyl, octadecenyl, nonadecenyl, eicosenyl, docosenyl, octadecadienyl, nonadecadienyl, eicosadienyl, octadecatrienyl, eicosatrienyl, eicosatetraenyl, octadecynyl, nonadecynyl, eicosynyl, and docosynyl, preferably from n-octanyl, n-nonanyl and 9Z-octadecenyl.

7. The use of Claim 5 characterized in that the compound has the general structure: 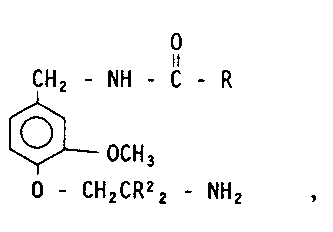 wherein -R is selected from n-hexanyl, n-heptanyl, n-octanyl, n-nonanyl, n-decanyl, n-undecanyl, n-dodecanyl, n-tridecanyl, tridecenyl, tetradecenyl, pentadecenyl, hexadecenyl, heptadecenyl, octadecenyl, nonadecenyl, eicosenyl, docosenyl, heptadecadienyl, octadecadienyl, nonadecadienyl, eicosadienyl, heptadecatrienyl, octadecatrienyl, nonadecatrienyl, eicosatrienyl, nonadecatetraenyl, heptadecynyl, octadecynyl, nonadecynyl, and eicosynyl, preferably from n-heptanyl, n-octanyl, and 8Z-heptadecenyl.

8. The use of Claim 7 characterized in that the compound is selected from N-((4-(2-methyl-2-aminopropoxy)-3-methoxyphenyl)-methyl)-9Z-octadecenamide and N-((4-(2-methyl-2-aminopropoxy)-3-methoxyphenyl)-methyl)-nonanamide.

9. The use of any of Claims 1-8 characterized in that said compound is administered topically to the genital region to prevent the recurrence of lesions.

10. The use of any of Claims 1-9 characterized in that the compound is used to treat herpes simplex Type II infections.

## Patentansprüche

1. Verwendung einer Verbindung zur Herstellung eines Medikaments zur Behandlung oder Verhütung von Vesikulareruptionen (Läsionen) infolge von Herpes simplex-Infektionen beim Menschen oder einem niedrigeren Tier, dadurch gekennzeichnet, daß die Verbindung die allgemeine Struktur: 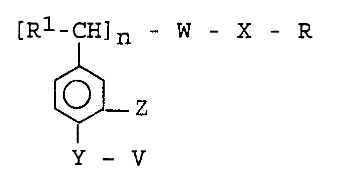 aufweist, worin:
a) n für 0 oder 1 steht, vorzugsweise 1 bedeutet;
b) -W-X- unter -C(O)NH-, -C(S)NH-, -S(O)₂NH-, -NHC(O)O-, -NHC(S)O-, -NHC(O)NH-, -NHC(S)NH-, -C(O)O-, -C(O)S- und -C(O)N(R)- ausgewählt ist; vorzugsweise unter -C(O)NH-, -C(S)NH-, -NHC(O)NH-, -NHC(S)NH- und -S(O)₂NH-; stärker bevorzugt unter -C(O)NH- und -C(S)NH-; worin eine verfügbare Bindung von -W-X- an -R gebunden ist und die andere Bindung an das Benzylkohlenstoffatom oder an den Benzolring gebunden ist;
c) -R¹ unter Wasserstoff, Hydroxy, Alkylestern von Hydroxy mit 1 bis 5 Kohlenstoffatomen, Alkyl mit 1 bis 5 Kohlenstoffatomen und Alkoxy mit 1 bis 5 Kohlenstoffatomen ausgewählt ist; vorzugsweise unter -H, -OH und -CH₃; stärker bevorzugt -H bedeutet;
d) -Z unter -H, -OH und -OCH₃ ausgewählt ist; bevorzugt unter -OH und -OCH₃; stärker bevorzugt -OCH₃ bedeutet;
e) -Y- unter -O-, -S-, -NH-, -OC(O)-, OSO₃²⁻- und -OPO₃²⁻-ausgewählt ist, vorzugsweise unter -O-, -S- und -NH-; stärker bevorzugt für -O- steht;
f) -V unter -H, Alkyl mit 1 bis 3 Kohlenstoffatomen und -CR²₂-CR²₂-NH₂ ausgewählt ist;
g) die Reste -R² unabhängig voneinander unter Wasserstoff, Halogen, unsubstituiertem oder substituiertem Alkyl mit 1 bis 6 Kohlenstoffatomen, substituiertem oder unsubstituiertem Aryl und Carboxylat ausgewählt sind, oder zwei Reste -R² covalent unter Ausbildung eines substituierten oder unsubstituierten Alkyl- oder Heteroalkylringes mit 3 bis 8 Atomen im Ring, einschließlich 0 bis 3 Heteroatomen, gebunden sind; und
h) -R unter C₂-C₂₄-Alkyl ausgewählt ist, das gerade, verzweigt oder cyclisch ist; gesättigt, einfach ungesättigt oder mehrfach ungesättigt ist; und substituiert oder unsubstituiert ist; oder zwei Reste -R covalent unter Ausbildung eines substituierten oder unsubstituierten Alkylringes mit 2 bis 14 Kohlenstoffatomen im Ring gebunden sind; wobei -R vorzugsweise unter unsubstituiertem, gesättigtem oder einfach oder zweifach ungesättigtem, geradkettigem oder verzweigtkettigem C₆-C₂₄-Alkyl ausgewählt ist;
oder die pharmazeutisch annehmbaren Salze und Amide hievon.

2. Verwendung nach Anspruch 1, dadurch gekennzeichnet, daß alle Reste -R² am alpha-Kohlenstoffatom -H bedeuten; beide Reste -R² am beta-Kohlenstoffatom unter -H, unsubstituiertem oder substituiertem C₁-C₆-Alkyl ausgewählt sind oder die Reste -R² covalent unter Ausbildung eines substituierten oder unsubstituierten C₃-C₆-Ringes gebunden sind, wobei vorzugsweise beide Reste -R² am beta-Kohlenstoffatom unter Methyl und Ethyl ausgewählt sind oder unter Ausbildung von Cyclopropyl, Cyclobutyl oder Cyclopentyl gebunden sind, stärker bevorzugt beide Reste Methyl darstellen; und -R für gesättigtes oder einfach oder zweifach durch Doppelbindungen ungesättigtes, gerades oder verzweigtkettiges C₆-C₂₄-Alkyl steht; vorzugsweise einfach ungesättigtes, geradkettiges C₁₁-C₂₃-Alkenyl mit cis-ständiger Doppelbindung oder geradkettiges C₇-C₁₀-Alkanyl bedeutet.

3. Verwendung nach Anspruch 2, dadurch gekennzeichnet, daß -V unter -H und -CH₃ ausgewählt ist.

4. Verwendung nach Anspruch 3, dadurch gekennzeichnet, daß diese Verbindung trans-8-Methyl-N-vanillyl-6-nonenamid oder N-Vanillylnonanamid ist.

5. Verwendung nach Anspruch 2, dadurch gekennzeichnet, daß -V für -CR²₂-CR²₂-NH₂ steht.

6. Verwendung nach Anspruch 5, dadurch gekennzeichnet, daß die Verbindung die allgemeine Struktur: 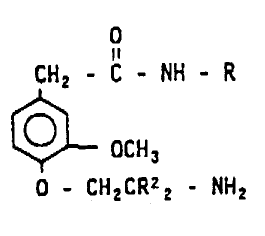 aufweist, worin -R unter n-Hexanyl, n-Heptanyl, n-Octanyl, n-Nonanyl, n-Decanyl, n-Undecanyl, n-Dodecanyl, n-Tridecanyl, n-Tetradecanyl, Tetradecenyl, Pentadecenyl, Hexadecenyl, Heptadecenyl, Octadecenyl, Nonadecenyl, Eicosenyl, Docosenyl, Octadecadienyl, Nonadecadienyl, Eicosadienyl, Octadecatrienyl, Eicosatrienyl, Eicosatetraenyl, Octadecinyl, Nonadecinyl, Eicosinyl und Docosinyl ausgewählt ist, vorzugsweise unter n-Octanyl, n-Nonanyl und 9Z-Octadecenyl.

7. Verwendung nach Anspruch 5, dadurch gekennzeichnet, daß die Verbindung die allgemeine Struktur: 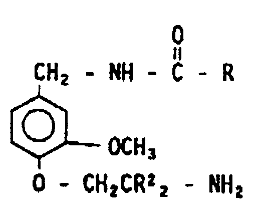 aufweist, worin -R unter n-Hexanyl, n-Heptanyl, n-Octanyl, n-Nonanyl, n-Decanyl, n-Undecanyl, n-Dodecanyl, n-Tridecanyl, Tridecenyl, Tetradecenyl, Pentadecenyl, Hexadecenyl, Heptadecenyl, Octadecenyl, Nonadecenyl, Eicosenyl, Docosenyl, Heptadecadienyl, Octadecadienyl, Nonadecadienyl, Eicosadienyl, Heptadecatrienyl, Octadecatrienyl, Nonadecatrienyl, Eicosatrienyl, Nonadecatetraenyl, Heptadecinyl, Octadecinyl, Nonadecinyl und Eicosinyl ausgewählt ist, vorzugsweise unter n-Heptanyl, n-Octanyl und 8Z-Heptadecenyl.

8. Verwendung nach Anspruch 7, dadurch gekennzeichnet, daß die Verbindung unter N-((4-(2-Methyl-2-aminopropoxy)-3-methoxyphenyl)-methyl)-9Z-octadecenamid und N-((4-(2-Methyl-2-aminopropoxy)-3-methoxyphenyl)-methyl)-nonanamid ausgewählt ist.

9. Verwendung nach einem der Ansprüche 1 bis 8, dadurch gekennzeichnet, daß diese Verbindung topisch auf den Genitalbereich zur Verhütung des Wiederauftretens von Läsionen verabreicht wird.

10. Verwendung nach einem der Ansprüche 1 bis 9, dadurch gekennzeichnet, daß die Verbindung zur Behandlung von Herpes simplex Typ II-Infektionen eingesetzt wird.

## Revendications

1. Utilisation d'un composé pour la fabrication d'un médicament à administrer localement, pour le traitement ou la prévention d'éruptions vésiculeuses (lésions) dues aux infections de l'herpès simplex chez un humain au un animal inférieur, caractérisée en ce que le composé passède la structure générale: 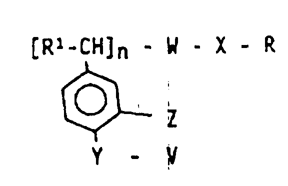 dans laquelle:
a) n est égal à 0 ou 1, de préférence égal à 1;
b) -W-X- est choisi parmi -C(O)NH-, -C(S)NH-, -S(O)₂NH-, -NHC(O)O-, -NHC(S)O-, -NHC(O)NH-, -NHC(S)NH-, -C(O)O-, -C(O)S-et -C(O)N(R)-; de préférence parmi -C(O)NH-, -C(S)NH-, -NHC(O)NH-, -NHC(S)NH-, et -S(O)₂NH-, et plus particulièrement parmi -C(O)NH-, et -C(S)NH-; où une liaison disponible de -W-X- est attachée à -R et l'autre liaison est attachée à l'atome de carbone benzylique ou au cycle benzénique;
c) -R¹ est choisi parmi un atome d'hydrogène, un hydroxy, des esters alxyliques d'hydroxy ayant de 1 à 5 atomes de carbone, un alkyle ayant de 1 à 5 atomes de carbone, et un alkoxy ayant de 1 à 5 atomes de carbone; de préférence parmi -H, -OH et -CH₃; et plus particulièrement -H;
d) -Z est choisi parmi -H, -OH, -OCH₃; de préférence parmi -OH et -OCH₃; et plus particulièrement -OCH₃;
e) -Y- est choisi parmi -O-, -S-, -NH-, -OC(O)-, -OSO₃²⁻-et -OPO₃²⁻-, de préférence parmi -O-, -S- et -NH-; et plus particulièrement -O-;
f) -V est choisi parmi -H, un alkyle en C₁ à C₃, et -CR²₂-CR²₂-NH₂;
g) les groupements -R² sont choisis indépendamment parmi, un atome d'hydrogène, un halogène, un alkyle substitué ou non ayant de 1 à 6 atomes de carbone, un aryle substitué ou non, et un carboxylate, ou deux groupements -R² sont liés de façon covalente pour former un cycle hétéroalkyle ou alkyle substitué ou non, ayant de 3 à 8 atomes dans le cycle dont notamment de 0 à 3 hétéroatomes; et
h) -R est choisi parmi un groupe alkyle en C₂-C₂₄ qui est linéaire, ramifié, ou à chaîne cyclique; saturé, mono-insaturé ou poly-insaturé; et substitué ou non substitué; ou deux groupements -R sont liés de façon covalente pour former un cycle alkyle substitué ou non, ayant de 2 à 14 atomes de carbone dans le cycle; de préférence -R est choisi parmi une chaîne alkyle en C₆-C₂₄ linéaire ou ramifiée, non substituée, saturée ou mono- ou di-insaturée; ou des sels et amides pharmaceutiquement acceptables de celui-ci.

2. Utilisation selon la revendication 1, caractérisée en ce que tous les groupements -R² sur le carbone α sont -H; les deux groupements -R² sur le carbone β sont choisis parmi -H, un alkyle en C₁-C₆ substitué ou non, ou les groupements - R² sont liés de façon covalente pour former un cycle en C₃-C₆ substitué ou non, de préférence les deux groupements -R² sur le carbone β sont choisis parmi les groupes méthyle et éthyle ou sont liés pour former un cyclopropyle, un cyclobutyle ou un cyclopentyle, de préférence les deux sont des groupes méthyle; et -R est un alkyle à chaîne en C₆-C₂₄ linéaire ou ramifiée saturée ou mono- ou di-insaturée par des doubles liaisons, de préférence un alkényle à chaîne linéaire en C₁₁-C₂₃ mono-insaturée, à double liaison cis ou un alkanyle à chaîne linéaire en C₇-C₁₀.

3. Utilisation selon la revendication 2, caractérisée en ce que -V est choisi parmi -H, et -CH₃.

4. Utilisation selon la revendication 3, caractérisée en ce que ledit composé est le trans-8-méthyl-N-vanillyl-6 nonénamide ou N-vanillylnonanamide.

5. Utilisation selon la revendication 2, caractérisée en ce que -V est -CR²₂-CR²₂-NH₂.

6. Utilisation selon la revendication 5, caractérisée en ce que le composé possède la structure générale: 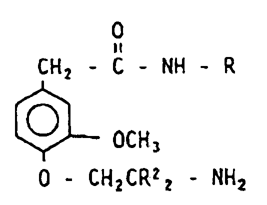 dans laquelle -R est choisi parmi le groupe n-hexanyle, n-heptanyle, n-octanyle, n-nonanyle, n-décanyle, n-undécanyle, n-dodécanyle, n-tridécanyle, n-tétradécanyle, tétradécényle, pentadécényle, hexadécényle, heptadécényle, octadécényle, nonadécényle, eicosényle, docosényle, octadécadiényle, nonadécadiényle, eicosadiényle, octadécatriényle, eicosatriényle, eicosatétraényle, octadécynyle, nonadécynyle, eicosynyle, et docosynyle, de préférence parmi le groupe n-octanyle, n-nonanyle et 9Z-octadécényle.

7. Utilisation selon la revendication 5, caractérisée en ce que le composé possède la structure générale: 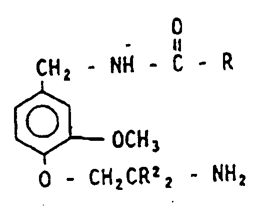 dans laquelle -R est choisi parmi le groupe n-hexanyle, n-heptanyle, n-octanyle, n-nonanyle, n-décanyle, n-undécanyle, n-dodécanyle, n-tridécanyle, tridécényle, tétradécényle, pentadécényle, hexadécényle, heptadécényle, octadécényle, nonadécényle, eicosényle, docosényle, heptadécadiényle, octadécadiényle, nonadécadiényle, eicosadiényle, heptadécatriényle, octadécatriényle, nonadécatriényle, eicosatriényle, nonadécatetraényle, heptadécynyle, octadécynyle, nonadécynyle et eicosynyle, de préférence parmi le groupe n-heptanyle, n-octanyle et 8Z-heptadécényle.

8. Utilisation selon la revendication 7, caractérisée en ce que le composé est choisi parmi le N-((4-(2-méthyl-2-aminopropoxy)-3-méthoxyphényl)-methyl)-9Z-octadécénamide et le N-((4-(2-méthyl-2-aminopropoxy)-3-méthoxyphényl)-méthyl)-nonanamide.

9. Utilisation selon l'une quelconque des revendications 1 à 8, caractérisée en ce que ledit composé est administré localement au niveau de la région génitale pour prévenir la récidive des lésions.

10. Utilisation selon l'une quelconque des revendications 1 à 9, caractérisée en ce que le composé est utilisé pour traiter les infections de l'herpès simplex du type II.
